Europäisches Patentamt

European Patent Office

Office européen des brevets

(11) Publication number : 0 515 216 A2

# (12) EUROPEAN PATENT APPLICATION

(21) Application number : 92304676.7

(22) Date of filing : 22.05.92

(51) Int. Cl.⁵ : C08B 37/00, // C12P19/04

(30) Priority : 24.05.91 JP 120204/91
09.03.92 JP 50968/92

(43) Date of publication of application :
25.11.92 Bulletin 92/48

(84) Designated Contracting States :
AT BE CH DE DK ES FR GB GR IT LI LU NL PT
SE

(71) Applicant : TAKEDA CHEMICAL INDUSTRIES,
LTD.
1-1, Doshomachi 4-chome
Chuo-ku, Osaka 541 (JP)

(72) Inventor : Haze, Akira
4-45, Seiwadainishi 5-chome
Kawanishi, Hyogo 666-01 (JP)
Inventor : Miyanagi, Kazuki
1025, Saikujo, Shikata-cho
Kakogawa, Hyogo 675 (JP)
Inventor : Tanaka, Yoshihiko
1-70, Fushimidai 2-chome, Inagawa-cho
Kawabe-gun, Hyogo 666-02 (JP)
Inventor : Sugiyama, Ryo
27-10, Tsukumodai 6-chome
Suita, Osaka 565 (JP)

(74) Representative : Lewin, John Harvey
Elkington and Fife Prospect House 8
Pembroke Road
Sevenoaks, Kent TN13 1XR (GB)

(54) Method of purifying beta-1,3-glucans.

(57)   This invention relates to a method of purifying β-1,3-glucans which comprises cooling a hot alkaline solution of a microbially produced β-1,3-glucan in a mixture of water and a hydrophilic organic solvent, then, where necessary, further adding a hydrophilic organic solvent, and neutralizing the mixture containing precipitated glucan.

The invention provides a method of purifying β-1,3-glucans which is commercially very advantageous in that it does not entail an excessive rise of viscosity and, hence, insures the ease of handling. The β-1,3-glucans purified by the method of the invention impart excellent thickening, water-holding, swelling and or plastic characteristics to various substrates and can be used advantageously in chemical, food and other industries.

EP 0 515 216 A2

Background of the Invention

This invention relates to a method of purifying β-1,3-glucans, which are useful in civil engineering, chemical, food and other industries.

There exist, in the natural kingdom, a number of β-1,3-glucan-producing microorganisms. For instance, curdlan is known to be a β-1,3-glucan produced extracellularly by certain microorganisms belonging to the genus Alcaligenes or Agrobacterium (New Food Industry, 20, 49, (1978); U.S. Patent No. 3,822,250).

As methods of purifying or producing β-1,3-glucans, for example, a method in which a β-1,3-glucan is treated with an aqueous alkali solution and, after separation of insolubles from the resulting solution, neutralizing the solution with acid to cause precipitation of the glucan (U.S. Patent No. 3,822,250); a method in which a hydrated gel or hydrated sol of hydrophilic macromolecular substances (e.g. curdlan) is frozen and then immersing and thawing the resulting frozen substances in a hydrophilic organic solvent (EP-A-343459); and a method in which a heat-nongelable β-1,3-glucan is treated with alkali and the solution is adjusted to a pH not exceeding pH 10 to cause precipitation of the heat-gelable β-1,3-glucan (EP-A-409488) have been reported.

Heat-gelable β-1,3-glucans such as curdlan develop high viscosity and, hence, are poor in workability when they are in the form of alkaline solutions or neutralized solutions in the process of purification. Therefore, these glucans must be handled in dilute solution forms, for instance, thus calling for very troublesome procedures.

Brief Description of the Drawings

Fig. 1 shows the relationship between the methanol charging ratio and the gel strength of the β-1,3-glucan powder.

Fig. 2 shows the relationship between the methanol charging ratio and the optical rotation of the β-1,3-glucan powder-derived DMSO supernatant.

Fig. 3 shows the relationship between the ethanol charging ratio and the gel strength of the β-1,3-glucan powder.

Fig. 4 shows the relationship between the ethanol charging ratio and the optical rotation of the β-1,3-glucan powder-derived DMSO supernatant.

Detailed Description

In view of the above circumstances, the inventors of the present invention made investigations to find out an industrially advantageous method of purifying β-1,3-glucan powders, which have viscosity buildup, water-holding and/or swelling characteristics, and, as a result, completed the present invention.

Thus the invention provides a method of purifying β-1,3-glucans which comprises cooling a hot alkaline solution of microbially produced β-1,3-glucans in a mixture of water and a hydrophilic organic solvent, then, where necessary, further adding a hydrophilic organic solvent, and neutralizing the mixture containing precipitated glucan.

The microorganism to be used in the practice of the invention may be any β-1,3-glucan-producing strain. Thus, for example, use can be made of microorganisms of the above-mentioned genus Alcaligenes or Agrobacterium, and euglenas, which are a kind of protozoa.

As protozoa of the genus Euglena, there may be mentioned, for example, Euglena gracilis and Euglena gracilis var. bacillaris.

As more specific examples, the following strains may be mentioned: Euglena gracilis Klebs NIES-47, Euglena gracilis Klebs NIES-48, and Euglena gracilis var. bacillaris Pringsheim NIES-49. These are known strains deposited with, and available from, the juridical foundation Chikyu Ningen Kankyo Forum (National Institute of Environmental Sciences).

Euglenas are ready to undergo mutation whether spontaneously or upon exposure to artificial mutagenic procedures such as X-ray irradiations, ultraviolet irradiation, emission-ray irradiation, and treatment with artificial mutagens. The resulting mutants, if capable of accumulating a β-1,3-glucan intracellularly, may also be used in the practice of the invention.

The β-1,3-glucans to be purified in accordance with the invention are prepared by cultivating microbial cells containing said glucans, for instance. The β-1,3-glucan used in this invention is, for instance, a polysaccharide containing glucose which is β-1,3-bonded and has degree of polymerisation of about 5 to 6,000, preferable degree of polymerisation of about 100 to 1,000. Examples of the β-1,3-glucan are curdlan, paramylon, pachyman, scleroglucan, laminalrin and yeast glucan. Curdlan is preferred. The above glucans may be an isolated product or may be used in the form of a culture broth. For operation efficiency reasons, culture broths are generally used.

EP 0 515 216 A2

When a culture broth is used, said culture broth may be used as such or after concentration using a centrifuge or after dilution with water.

The alkali is not limited to any specific species but, generally, an alkali metal hydroxide such as sodium hydroxide or potassium hydroxide, or ammonium hydroxide is preferred.

The concentration of the alkali is the hot alkaline solution of β-1,3-glucans in a mixture of water and a hydrophilic organic solvent is required only to be such that the β-1,3-glucans can be apparently dissolved. For instance, when a β-1,3-glucan produced by an Alcaligenes strain is used, a concentration of not less than 0.2 N is sufficient. Generally, a concentration of about 0.2 to 1 N, preferably about 0.3 to 0.5 N, is employed.

The hydrophilic organic solvent to be used in the practice of the invention includes alcohols such as methyl alcohol (methanol), ethyl alcohol (ethanol) and isopropyl alcohol, ketones such as acetone and methylethylketone, and nitriles such as acetonitrile and propionitrile. Alcohols are preferred, and methanol and ethanol are more preferred. Methanol is more specifically preferred.

The hot alkaline solution of a microbially produced β-1,3-glucan in a mixture of water and a hydrophilic organic solvent, which is to be treated in accordance with the invention, can be prepared, for example, (1) by mixing an alkali and a hydrophilic organic solvent with an aqueous dispersion of β-1,3-glucans and heating the resulting mixture, or (2) by adding an alkali to an aqueous dispersion of β-1,3-glucans , heating the mixture, and then adding a hydrophilic organic solvent to the mixture, or (3) by adding an alkali to an aqueous dispersion of β-1,3-glucans , and then adding a hydrophilic organic solvent to the mixture with heating the mixture. Thus, it is only required that an alkaline hot solution of β-1,3-glucans in a mixture of water and a hydrophilic organic solvent should be in hand prior to the cooling operation in the next step. Among the methods mentioned just above, the one comprising mixing an alkali and a hydrophilic organic solvent with an aqueous dispersion of β-1,3-glucans and heating the resulting mixture is preferred. Said heating may be conducted in a mixing apparatus such as a stirring vessel or a kneader.

The above mixed solution is heated to a temperature of not lower than 40°C, generally about 40° to 80°C. About 50° to 65°C is preferred.

When alcohols are used as the hydrophilic organic solvent in the above-mentioned mixed solution, the alcohols are suitably used in an amount of 0.1 to 3 volumes per volume of water in said hot solution. Thus, methanol, for instance, is preferably used in an amount of about 0.2 to 3, more preferably about 0.2 to 2.5, volumes per volume of water in the hot solution. More specifically, the lower limit for methanol is about 0.2 volume, desirably about 0.3 volume, per volume of water. At methanol levels below 0.2 volume, the operation in the step of heating for dissolution will be difficult to perform. The upper limit is about 2.5 volumes, desirably about 1 volume, more desirably about 0.6 volume, per volume of water.

The mixed solvent solution is then cooled to a temperature around room temperature, generally about 10° to 30°C, preferably about 15° to 25°C.

After cooling, an additional amount of a hydrophilic organic solvent may be added to promote the dehydration/separation in the centrifugal dehydration step to be mentioned later herein. Said additional amount is from about 0.5 volume, preferably from about 1.2 volumes, to about 5 volumes per volume of the initial β-1,3-glucan-containing aqueous solution, although there is no particular upper limit.

The precipitated glucan-containing mixture is then neutralized to a pH of about 8 or below. Considering the stability of β-1,3-glucans, the pH after neutralization should preferably be about 5 to 7, more preferably about 5 to 6.

While any pH adjusting agent may suitably be employed, inorganic acids are generally used. Mineral acids such as hydrochloric acid are preferred.

From the industrial use viewpoint, it is advantageous that the above neutralized β-1,3-glucan precipitate-containing mixture (β-1,3-glucan slurry) be subjected, after desalting, concentration and, further drying treatment, to pulverization to give a readily handleable powder. Thus, for instance, the β-1,3-glucan slurry obtained in the above manner is subjected to dehydration by centrifugation. The dehydration product (solid β-1,3-glucan content 10 to 30% w/w) is further brought into contact with an alcohol, acetone or the like for dehydration with the solvent and again subjected to centrifugal dehydration to give a dehydration product (solid β-1,3-glucan content 30 to 50% w/w) which can be dried more easily. This is then dried under reduced pressure and the solids obtained are ground to give a dried powder.

β-1,3-Glucans obtained by the method of the invention show a solubility of not less than 3% in cement filtrates. On the contrary, with β-1,3-glucans obtained by subjecting the culture broth directly to spray drying (using hot air at 200°), the solubility in cement filtrates is generally about 1% and at most less than 3%.

The term "solubility in cement filtrates" as used herein is now explained. Ordinary portland cement (50 g), 50 ml of water and 1 g of a β-1,3-glucan powder are mixed for 30 minutes and then centrifuged. The supernatant is assayed for total sugar concentration (A %) by the phenol-sulfuric acid method. The solubility is calculated as follows:

3

$$\text{Solubility} = [(50 \text{ (g)} \times \frac{A(\%)}{100} \div (1 \text{ g})] \times 100$$

$$= 50 \text{ A } (\%) \qquad [I]$$

Among β-1,3-glucan powders obtainable by the method of the invention, those showing a degree of solubilization of about 3 to 30%, preferably about 3 to 6%, in cement filtrates can be used in various fields of civil engineering and chemical industry. For instance, they can be used as rheology modifiers for solutions and pastes, like methylcellulose. β-1,3-glucans purified by the method of this invention all can be used in the food industry (e.g. animal meat products and pasty foods based on fish flesh or animal meat, such as steamed or backed fish paste, animal meat hams such as boneless ham, roast ham, etc., fish hams, mixed fish and animal meat ham, sausages including Vienna sausage, corned beef, hamburg steak, etc., artificial meats based on soybean or wheat protein, soybean curd curd, miso, sauces, ketchups, tomato juice, nectar drinks, juices, jams, marmalades, peanut butter, flour paste, bean jams, soy-cooked delicacies such as cooked beans, various gourmet foods, gravies, instant curry mixes, instant stews, instant soups, butter margarine, cheese, syrups, candied fruits, textured protein, soy protein, spun protein, noodles, soba noodles, Chinese noodles, rice noodles, macaroni, spaghetti, and other cereal flour foods, rice cake, boiled rice foods, confectioners' steamed rice, steamed rice for the production of sake , steamed rice for the production of miso, steamed wheat and other like cereals for cooking use, candies cookies, fried cakes, bavarois, marshmallows, cream puffs, corn cups and wafers for frozen confections and the like.). When they are used for food, the hydrophilic organic solvent is preferably ethanol.

The β-1,3-glucan slurry obtained by the method of the invention which uses a hydrophilic organic solvent has a lower viscosity as compared with the corresponding slurry obtained by neutralization for β-1,3-glucan precipitation without using any hydrophilic organic solvent and may also be regarded as a slurry in the hydrophilic organic solvent. Therefore said slurry can be readily dehydrated by centrifugation and this renders the purification method of the invention very advantageous from the commercial production viewpoint.

β-1,3-glucans varying in heat-gelability can be produced and purified by the purification method of this invention. For obtaining highly heat-gelable (high gel strength) products, sufficient dissolution of β-1,3-glucans is required. As regards this dissolution, the following have been established in general: a β-1,3-glucan can be dissolved more readily when its concentration in the solution is lower, the alkali concentration is higher, the heating temperature is higher, the heating time is longer and the level of addition of the hydrophilic organic solvent is lower. However, excessive dissolution causes alkaline degradation of β-1,3-glucan polymers and this results in a decreased gel strength. Therefore, the above conditions should be selected to be suitable.

The invention provides a method of purifying β-1,3-glucans which is commercially very advantageous in that it does not entail an excessive rise of viscosity and, hence, insures the ease of handling. Furthermore, β-1,3-glucans purified in accordance with the invention impart excellent thickening, water-retaining, swelling and/or plasticity characteristics to various substrates and can be used advantageously in various chemical, food and other industries.

The following working examples illustrate the invention in further detail.

Example 1

To 500 ml of a culture broth obtained with Alcaligenes faecalis var. myxogenes NTK-u (ATCC 21680) in the conventional manner [β-1,3-glucan (curdlan) content about 4% w/w] was added 20.8 ml of 10 N sodium hydroxide to adjust the alkali concentration of the culture broth to 0.4 N sodium hydroxide. This procedure was repeated with another 500-ml portion of the culture broth. The two alkaline solutions each derived from the culture broth were heated to 50°C and 60°C, respectively, for 2 hours to give the respective hot solutions.

Methanol (referred to as "methanol after dissolution" in Table 1) was added to the respective solutions having the above sodium hydroxide concentration (0.4 N), in an amount of 0.5 volume (i.e. 250 ml), 0.75 volume (i.e. 375 ml) or 1 volume (i.e. 500 ml), under cooling and stirring of the solutions (the temperature after cooling: 20°C). Then, methanol (referred to as "methanol after cooling" in Table 1) was added in the amounts specified in Table 1 (i.e. 750 ml, 625 ml, 500 ml), which was followed by neutralization with 12 N hydrochloric acid.

Each neutralizate was subjected to dehydration in a basket centrifuge, 1,000 ml of methanol was added to the dehydration product, and the mixture was subjected to solvent dehydration in the same basket centrifuge. The dehydration product obtained was dried in vacuo at 60°C to give a β-1,3-glucan powder.

When evaluated by the methods mentioned below, the β-1,3-glucans obtained in the above manner gave the results shown in Table 1.

Methods of evaluation

(1) Gel strength

A β-1,3-glucan sample (0.2 g) was placed in a homogenizer, 10 ml of water was added, and the mixture was allowed to stand for 30 minutes. Then the mixture was stirred using a small-sized motor-driven agitator (2,000 rpm) for 5 minutes. The resultant suspension (2% aqueous dispersion of the β-1,3-glucan powder) was transferred to a test tube, deaerated under reduced pressure, and then, heated in a boiling water bath for 10 minutes. The suspension was cooled in running water for 10 minutes and then allowed to stand at room temperature for 30 minutes. Then, test specimens, 10 mm long, were prepared and measured for gel strength using a curd meter.

(2) Degree of swelling in water

A 2% aqueous dispersion of a β-1,3-glucan suspension was prepared as described above under (1). This aqueous dispersion was placed in a 100-ml graduated cylinder and the volume of the swollen β-1,3-glucan powder was measured.

The degree of swelling in water was expressed in terms of the ratio:

$$\frac{(\text{Volume of swollen } \beta - 1,3 - \text{glucan powder: ml})}{(\text{Volume of } \beta - 1,3 - \text{glucan powder before addition of water: ml})}$$

(3) Degree of swelling in cement filtrates

Water (90 g) was added to 100 g of ordinary portland cement, the mixture was stirred for 1 hour, and this cement paste was filtered through a filter paper (No. 2) under reduced pressure. A β-1,3-glucan powder was added to the filtrate (cement filtrate) obtained to a concentration of 1% (w/w). The resultant mixture was placed in a 100-ml graduated cylinder and the volume of the swollen β-1,3-glucan was measured. The degree of swelling in cement filtrates was expressed in terms of the ratio:

$$\frac{(\text{Volume of swollen } \beta - 1,3 - \text{glucan powder: ml})}{(\text{Volume of } \beta - 1,3 - \text{glucan powder before addition to the filtrate: ml})}$$

(4) Mortar flow value

Ordinary portland cement (100 g), 100 g of blast furnace slag, 133.3 g of fly ash, 465.3 g of sand (for testing purposes), 107 g of water, 5 g of a high-performance water-reducing agent and 1 g of β-1,3-glucan powder were blended in a universal mixer for 2 minutes and then the mortar flow value was determined on a JIS mortar flow meter.

(5) Solubility in cement filtrates

Ordinary portland cement (50 g), 50 ml of water and 1 g of β-1,3-glucan powder were blended for 30 minutes, the mixture was then centrifuged, and the total sugar concentration of the supernatant was determined by the phenol-sulfuric acid method. The solubility was calculated using the above formula [I].

Table 1

| | Conditions of heating for dissolution | | | | | |
|---|---|---|---|---|---|---|
| | 60°C, 2 hours | | | 50°C, 2 hours | | |
| Amount of methanol after dissolution (ml) | 500 | 375 | 250 | 500 | 375 | 250 |
| Amount of methanol after cooling (ml) | 500 | 625 | 750 | 500 | 625 | 750 |
| Characteristics of lyophilizate — Lyophilizate (g) | 17.2 | 18.1 | 18.0 | 17.3 | 18.3 | 18.2 |
| Gel strength (g/cm$^2$) | 790 | 918 | 902 | 634 | 798 | 713 |
| Degree of swelling in water | 5.2 | 6.0 | 5.8 | 4.9 | 5.4 | 5.5 |
| Degree of swelling in cement filtrate | 10.8 | 13.2 | 12.5 | 9.8 | 12.8 | 11.5 |
| Mortar flow value (cm) | 15.6 | 14.5 | 14.5 | 18.0 | 15.2 | 15.5 |
| Solubility in cement filtrate(%) | 30 | 35 | 40 | 20 | 25 | 30 |

## Example 2

10 N Sodium hydroxide was added to 500-ml portions of the same culture broth as used in Example 1 in three amounts indicated in Table 2 to adjust the culture broth portions to an alkalinity of 0.4 N sodium hydroxide with simultaneous addition of methanol (referred to as "methanol before dissolution" in Table 2) in amounts of 0.5 volume (i.e. 250 ml), 0.6 volume (i.e. 300 ml) and 0.75 volume (i.e. 375 ml), respectively, per volume of the alkaline broth. This procedure was performed in pairs. For each pair, one β-1,3-glucan-containing alkaline water-hydrophilic organic solvent mixture was heated at 50°C, and the other at 60°C, for 2 hours. The solutions thus obtained were cooled, whereupon fine crystals appeared at 30° to 40°C. After cooling to 20°C, methanol (referred to as "methanol after cooling" in Table 2) was added in the respective amounts indicated in Table 2 (750 ml, 625 ml and 500 ml), followed by neutralization using 12 N hydrochloric acid.

Each neutralizate was subjected to centrifugal dehydration and vacuum drying in the same manner as in Example 1 to give a β-1,3-glucan powder. The powder was evaluated in the same manner as in Example 1. The results thus obtained are shown in Table 2.

## Table 2

|  |  | 50°C, 2 hours | | | 60°C, 2 hours | | |
|---|---|---|---|---|---|---|---|
|  | Conditions of heating for dissolution | | | | | | |
|  | Amount of 10 N NaOH added (ml) | 31.5 | 33.1 | 36.5 | 31.5 | 33.1 | 36.5 |
|  | Amount of methanol before dissolution (ml) | 250 | 300 | 375 | 250 | 300 | 375 |
|  | Amount of methanol after cooling (ml) | 750 | 700 | 625 | 750 | 700 | 625 |
|  | Lyophilizate (g) | 19.3 | 19.4 | 18.7 | 18.4 | 19.3 | 19.4 |
| Characteristics of lyophilizate | Gel strength (g/cm$^2$) | 0 | 149 | 105 | 574 | 474 | 402 |
|  | Degree of swelling in water | 5.6 | 5.2 | 4.3 | 6.0 | 5.6 | 4.5 |
|  | Degree of swelling in cement filtrate | 13.7 | 13.0 | 10.5 | 14.1 | 13.6 | 11.5 |
|  | Mortar flow value (cm) | 24.5 | 25.0 | 26.5 | 22.5 | 23.5 | 25.0 |
|  | Solubility in cement filtrate (%) | 5.0 | 4.0 | 3.2 | 6.8 | 6.8 | 6.5 |

Example 3

10 N Sodium hydroxide was added to 500-ml portions of the same culture broth as used in Example 1 in three amounts indicated in Table 3 to give sodium hydroxide concentrations of 0.4 N, 0.6 N and 0.8 N upon simultaneous addition of methanol in 375-ml portions, followed by heating at 60°C for 2 hours for effecting dissolution. The resultant solutions were cooled, whereupon fine crystals appeared at 30° to 40°C. After cooling to 20°C, methanol was added in 625-ml portions, followed by neutralization with 12 N hydrochloric acid.

Each neutralizate was subjected to centrifugal dehydration and vacuum drying in the same manner as in Example 1 to give a β-1,3-glucan powder, which was evaluated in the same manner as in Example 1. The results thus obtained are shown in Table 3.

### Table 3

| | | | |
|---|---|---|---|
| Alkari concentration (N) | 0.4 | 0.6 | 0.8 |
| Amount of 10 N NaOH added (ml) | 36.5 | 56.0 | 76.0 |
| Gel strength ($g/cm^2$) | 402 | 810 | 1042 |
| Degree of swelling in water | 6.1 | 5.7 | 5.0 |
| Degree of swelling in cement filtrate | 13.5 | 13.5 | 12.8 |

### Example 4

Methanol (hereinafter referred to as "methanol before dissolution" in Table 4 and elsewhere) was added to 500-ml portions of the same culture broth as used in Example 1 in the amounts indicated in Table 4, namely 0.2 volume (i.e. 100 ml), 0.3 volume (i.e. 150 ml), 0.4 volume (i.e. 200 ml), 0.5 volume (i.e. 250 ml), 0.6 volume (i.e. 300 ml), 0.75 volume (i.e. 375 ml) and 1.0 volume (i.e. 500 ml) per volume of said culture broth. After stirring uniformly, sodium hydroxide was added in the respective amounts indicated in Table 4 to adjust the sodium hydroxide concentration of each β-1,3-glucan -containing water-hydrophilic organic solvent mixture to 0.4 N. After 2 hours of heating at 60°C for effecting dissolution, the solutions obtained were cooled, whereupon fine crystals appeared at 20° to 30°C. After cooling to 15°C, methanol was added in the amounts indicated in Table 4, followed by neutralization with 12 N hydrochloric acid.

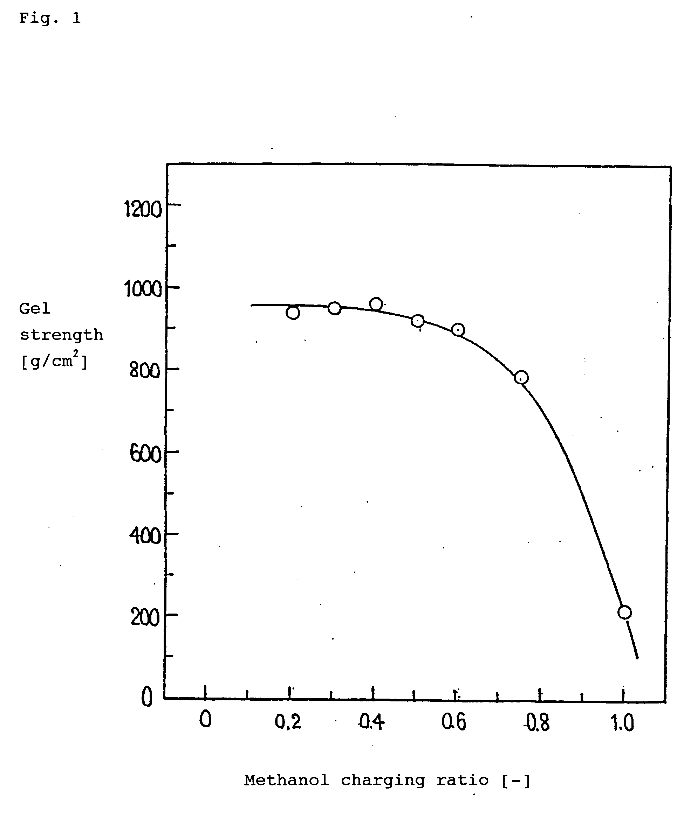

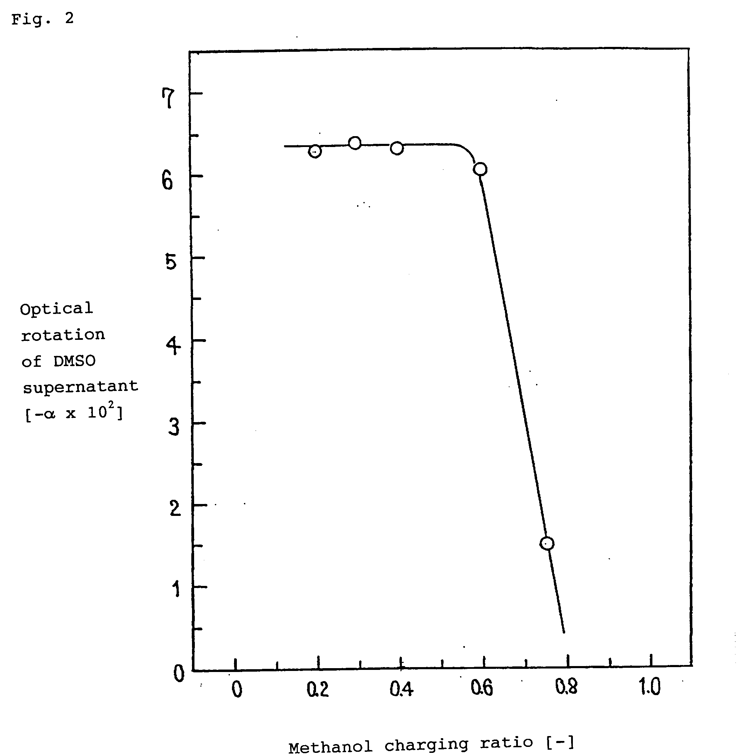

Each neutralizate was subjected to centrifugal dehydration and vacuum drying in the same manner as in Example 1 to give a β-1,3-glucan powder. For this powder, the gel strength and the solubility in dimethyl sulfoxide (DMSO) were measured. The results thus obtained are shown in Fig. 1 and Fig. 2.

In Figs 1 and 2, the methanol charging ratio is defined by the formula:

$$\frac{\text{(Amount of methanol before dissolution: ml)}}{\text{(500 ml of culture broth)}}$$

From Fig. 1, it is seen that when the methanol charging ratio is not higher than 0.8, the β-1,3-glucan powder has a high gel strength.

From Fig. 2, it is seen that when the methanol charging ratio is not higher than 0.6, the β-1,3-glucan powders are very soluble in DMSO.

### Methods of evaluation

#### (1) Gel strength

The same evaluation procedure was followed as in Example 1.

#### (2) Solubility in dimethyl sulfoxide (DMSO)

Each β-1,3-glucan sample was added to DMSO, in an amount to give a concentration of 0.5% (w/w). After 30 minutes of stirring, the mixture was centrifuged and the optical rotation of the supernatant was measured. The optical rotation of the DMSO supernatant was used as an index of solubility in DMSO. (The higher the optical rotation value, the higher the solubility of the β-1,3-glucan.)

Table 4

| Amount of methanol before dissolution (ml) | 100 | 150 | 200 | 250 | 300 | 375 | 500 |
| Methanol charging ratio | 0.2 | 0.3 | 0.4 | 0.5 | 0.6 | 0.75 | 1.0 |
| Amount of 10 N NaOH added (ml) | 25.0 | 27.1 | 29.2 | 31.3 | 33.3 | 36.5 | 41.7 |
| Amount of methanol after cooling (ml) | 900 | 850 | 800 | 750 | 700 | 625 | 500 |

Example 5

500 ml of the culture broth as used in Example 1 is diluted with 750 ml of water to adjust the β-1,3-glucan (curdlan) content to about 1.6% w/w.

Ethyl alcohol (hereinafter referred to as "ethanol before dissolution" in Table 5 and elsewhere) was added to 1250-ml portions of the diluted culture broth as mentioned above in the amounts indicated in Table 5, namely 0.1 volume (i.e. 125 ml), 0.2 volume (i.e. 250 ml), 0.3 volume (i.e. 375 ml), 0.4 volume (i.e. 500 ml), 0.5 volume (i.e. 625 ml), 0.6 volume (i.e. 750 ml), 0.7 volume (i.e. 875 ml) and 0.75 volume (i.e. 938 ml) per volume of said culture broth. After stirring uniformly, sodium hydroxide was added in the respective amounts indicated in Table 4 to adjust the sodium hydroxide concentration of each β-1,3-glucan-containing water-hydrophilic organic solvent mixture to 0.4 N. After 2 hours of heating at 60°C for effecting dissolution, the solutions obtained were cooled, whereupon fine crystals appeared at 20° to 30°C. After cooling to 15°C, ethanol was added in the amounts indicated in Table 5, followed by neutralization with 12 N hydrochloric acid.

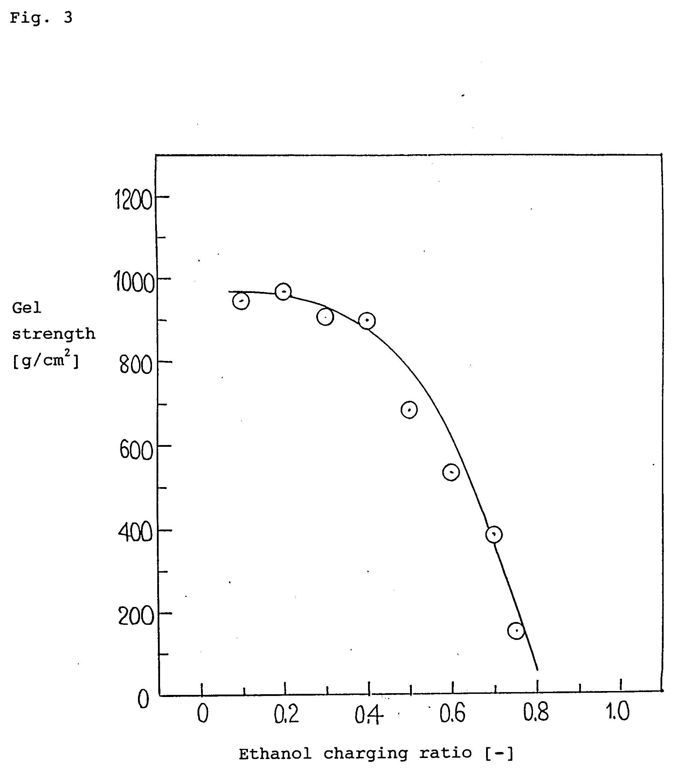

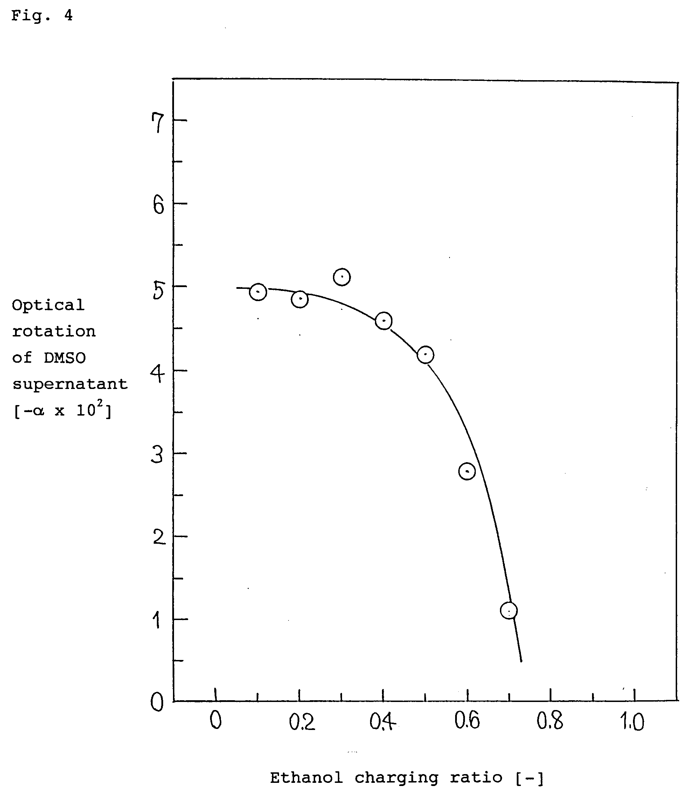

Each neutralizate was subjected to centrifugal dehydration and vacuum drying in the same manner as in Example 1 to give a β-1,3-glucan powder. For this powder, the gel strength and the solubility in dimethyl sulfoxide (DMSO) were measured in the same manner as in Example 4. The results thus obtained are shown in Fig. 3 and Fig. 4.

In Figs 3 and 4, the ethanol charging ratio defined by the formula:

$$\frac{\text{(Amount of methanol before dissolution:ml)}}{\text{(1250 ml of culture broth)}}$$

From Fig. 3, it is seen that when the ethanol charging ratio is not higher than 0.4, the β-1,3-glucan powder has a high gel strength.

From Fig. 4, it is seen that when the ethanol charging ratio is not higher than 0.4, the β-1,3-glucan powders are very soluble in DMSO.

Table 5

| | | | | | | | | |
|---|---|---|---|---|---|---|---|---|
| Amount of ethanol before dissolution (ml) | 125 | 250 | 375 | 500 | 625 | 750 | 875 | 938 |
| Ethanol charging ratio | 0.1 | 0.2 | 0.3 | 0.4 | 0.5 | 0.6 | 0.7 | 0.75 |
| Amount of 10 N NaOH added (ml) | 55 | 60 | 65 | 70 | 75 | 80 | 85 | 87.5 |
| Amount of ethanol after cooling (ml) | 2375 | 2250 | 2125 | 2000 | 1875 | 1750 | 1625 | 1562 |

**Claims**

1. A method of purifying β-1,3-glucans which comprises cooling a hot alkaline solution of a microbially produced β-1,3-glucans in a mixture of water and a hydrophilic organic solvent, then, where necessary, further adding a hydrophilic organic solvent, and neutralizing the mixture containing precipitated glucan.

2. The method as claimed in claim 1, wherein an alkali and a hydrophilic organic solvent are mixed with an aqueous dispersion of β-1,3-glucans, and the resulting mixture is heated to obtain a hot alkaline solution of a microbially produced β-1,3-glucans in a mixture of water and a hydrophilic organic solvent.

3. The method as claimed in claim 1, wherein the β-1,3-glucans are curdlan.

4. The method as claimed in claim 1, wherein the hydrophilic organic solvent is an alcohol.

5. The method as claimed in claim 4, wherein the alcohol is methanol.

6. The method as claimed in claim 5, wherein the ratio of methanol to water in the hot alkaline solution is about 0.2 to 2.5 volume/volume.

Fig. 1

Fig. 2

Fig. 3

Fig. 4